# EUROPÄISCHE PATENTANMELDUNG

(11) **EP 1 232 723 A1**
(43) Veröffentlichungstag der Anmeldung: **21.08.2002**
(21) Anmeldenummer: 02003010.2
(22) Anmeldetag: 12.02.2002
(51) Int. Cl.: A61B 3/16

(54) **Intraokulares Implantat zur Messung des intraokularen Druckes**

(30) Priorität: 14.02.2001 DE 10106881
(71) Anmelder: Campus Micro Technologies GmbH, 28359 Bremen (DE)
(72) Erfinder: Draeger, J., Prof. Dr., Martinstrasse 52, 20246 Hamburg (DE); Eggers, Torsten, 28359 Bremen (DE); Benecke, Wolfgang, Prof. Dr., 28359 Bremen (DE)

(57) **Zusammenfassung**

Es wird ein intraokulares Implantat zurtelemetrischen Bestimmung des Innendrucks des menschlichen Auges angegeben, welches einen Grundkörper aus einem biokompatiblen Material und Telemetrieeinrichtungen zur Bestimmung und Abgabe eines den Augeninnendruck kennzeichnenden elektrischen Telemetriesignals enthält. Der Grundkörper besitzt eine längliche Form und hat in Längsrichtung einander gegenüberliegende Enden, zwischen denen sich eine Anlagefläche erstreckt, die als Segment einer Kugeloberfläche ausgebildet und entsprechend gekrümmt ist.

## Beschreibung

Die Erfindung betrifft ein intraokulares Implantat zur telemetrischen Bestimmung des Innendrucks des menschlichen Auges, mit einem Drucksensor zur Abgabe eines den Augeninnendruck kennzeichnenden elektrischen Sensorsignals, einer Telemetrieschaltung zur Erzeugung eines den Augeninnendruck kennzeichnenden Telemetriesignales aus dem Sensorsignal, einer Sende- und Empfangsspule zum drahtlosen Empfang von Speisesignalen, welche der Telemetrieschaltung ausreichend Speiseenergie zuführen, und zur drahtlosen Abgabe des Telemetriesignales an externe Empfangseinrichtungen, und einem Grundkörper aus einem biokompatiblen Material, der den Drucksensor, die Telemetrieschaltung und die Sende- und Empfangsspule trägt.

Das Glaukom, umgangssprachlich "Grüner Star", führt langfristig zur Erblindung und ist in den Industriestaaten die häufigste Erblindungsursache. Die Erkrankung ist nach heutigem Kenntnisstand ursächlich mit einem erhöhten intraokularen Druck korreliert. Dieser intraokulare Druck (IOD) wird bisher im Rahmen von Untersuchungen beim Augenarzt gelegentlich gemessen, um im Frühstadium eine mögliche Glaukom-Tendenz zu diagnostizieren. Nach erfolgter Diagnose wird der erhöhte intraokulare Druck medikamentös behandelt und regelmäßig ambulant oder bei kritischem Verlauf auch stationär gemessen. Überschreitet dieser Druck trotz medikamentöser Behandlung einen Schwellenwert, so kann in der Regel dieser Erkrankung nur durch eine Operation begegnet werden. Die derzeit nur geringe Meßdichte bei Glaukom-Patienten führt zu einer Unsicherheit bezüglich des Verlaufs der Krankheit, da der intraokulare Druck starke Schwankungen über den Tag aufweist. Aus der DE 198 58 172 ist ein Implantat zur Messung des Augeninnendrucks bekannt, welches eine künstliche Linse aufweist, die mit einem umlaufenden Randwulst im Auge befestigbar ist, wobei ein Drucksensor, eine Telemetrieschaltung und eine Sende- und Empfangsspule auf dem Randwulst um die künstliche Linse herum angeordnet ist und die vom Drucksensor abgetasteten Werte des Augeninnendruckes es in Telemetriesignale umwandeln, welche über die Sendeund Empfangsspule einem äußeren Lesegerät zugeführt werden. Bei diesem bekannten Implantat werden die Telemetrieeinrichtungen also in die bekannten künstlichen Linsen eingebracht und zusammen mit diesen künstlichen Linsen dann in das Auge einoperiert. Dabei ist es nachteilig, dass die intraokulare Druckmessung nur in Verbindung mit dem Austausch der natürlichen Linse durch eine künstliche Linse möglich ist und somit die Anwendung auf die Gruppe der Kataraktpatienten mit einer Glaukomkoinzidenz beschränkt bleibt.

Aus der DE 43 41 903 A1 ist ferner ein implantierbares telemetrisches Endosystem bekannt, welches u.a. zur kontinuierlichen Messung eines Drucks in einem menschlichen Körper geeignet ist. Die Vorrichtung besitzt einen Drucksensor, eine dem Drucksensor nachgeschaltete Signalverarbeitungsschaltung zur Umwandlung des Sensorsignals in ein Telemetriesignal, und die Telemetrieeinrichtung gibt dann diese Telemetriesignale an eine außerhalb des Körpers befindliche Leseeinheit ab, welche die Telemetriesignale verarbeitet und zur Anzeige bringt.

Aufgabe der Erfindung ist es, ein Implantat der eingangs genannten Art derart weiterzubilden, dass es ohne Austausch der natürlichen Linse in einer einfachen Operation in das menschliche Auge einsetzbar ist und den Augeninnendruck kennzeichnende Telemetriesignale nach außen an externe Empfangseinrichtungen abgibt.

Diese Aufgabe wird bei dem intraokularen Implantat der eingangs genannten Art erfindungsgemäß dadurch gelöst, dass der Grundkörper eine längliche Form und in Längsrichtung einander gegenüberliegende Enden besitzt, und dass ein Abschnitt, der sich zwischen den Enden erstreckenden Umfangsfläche des Grundkörpers in Längsrichtung mit einem Krümmungsradius R gekrümmt ist, und als Anlagefläche bezeichnet ist, wobei der Krümmungsradius R dem Krümmungsradius der inneren Oberfläche der Lederhaut des Auges entspricht.

Die Vorteile der Erfindung liegen insbesondere darin, dass das erfindungsgemäße Implantat unabhängig von anderen durchzuführenden operativen Eingriffen am Auge eine permanente berührungsfreie, noninvasive Messung des intraokularen Druckes ermöglicht, wenn das Implantat an der Innenfläche der Lederhaut, zwischen den Zonular-Fäden und der Netzhaut an der als "Pars plana" bezeichneten Stelle des menschlichen Auges operativ befestigt wird. Die Telemetrieeinrichtungen, welche das Sensorsignal berührungsfrei nach außen einer externen Empfangseinrichtung zuführen, sind erfindungsgemäß in ein Implantat integriert, welches ausschließlich den Zweck hat, den intraokularen Druck zu messen oder festzustellen. Diese Telemetrieeinrichtungen sind erfindungsgemäß also nicht in einem künstlichen Linsenimplantat integriert. Dadas erfindungsgemäße Implantat nur dem Ziel dient, den intraokularen Druck zu messen, kann das Implantat besonders klein, in miniaturisierter Bauweise, ausgeführt werden und lässt sich mit einem minimalisierten operativen Eingriff in den Augeninnenraum einführen und dann an der Innenseite der Lederhaut - beispielsweise mittels geeigneter Nähte anbringen. Dabei ist derjenige Umfangsabschnitt der Oberfläche des Implantats, der in Längsrichtung des Grundkörpers gekrümmt ist oder besonders bevorzugt sogar als Kugelflächensegment ausgebildet ist, mit einer Krümmung versehen, die etwa der Krümmung der Innenfläche der Lederhaut entspricht, so dass das Implantat beim Einoperieren im wesentlichen formschlüssig an der Innenfläche der Lederhaut - zwischen der Netzhaut - und den Zonular-Fasern, welche die Linse halten, anliegt. Auf diese Weise wird sichergestellt, dass das Implantat einfach einzuoperieren ist und dann mit einer vergleichsweise großen Oberfläche an der Innenfläche der Lederhaut anliegt.

Besonders bevorzugt ist die Anlagefläche des Implantats als Segment einer Kugeloberfläche ausgebildet, deren Radius R dem Krümmungsradius der Innenfläche der Lederhaut des menschlichen Auges entspricht, damit eine besonders formschlüssige Anlage des Implantats an derjenigen Stelle des Auges erreicht wird, an der das Implantat operativ befestigt werden soll.

Der Krümmungsradius des Flächenabschnitts des Implantats, der - nach dem Einoperieren - an der Innenfläche der Lederhaut des menschlichen Auges anliegt, entspricht im wesentlichen dem Krümmungsradius dieser Innenfläche der Lederhaut und liegt zwischen 10 mm und 12 mm, näherungsweise bei 11 mm.

Um ein vergleichsweise einfaches Einführen und Befestigen des Implantates an der als "Pars plana" bezeichneten Stelle an der Innenfläche der Lederhaut zu erleichtern, ist der längliche Grundkörper stabförmig ausgebildet und verjüngt sich zu beiden Enden von einer mittigen Querschnittsebene näherungsweise symmetrisch. Der Grundkörper läuft im wesentlichen spitz zu seinen Enden hin zu, die Enden sind abgerundet, um ein einfaches Einführen durch eine entsprechend klein bemessenene Operationsöffnung durch die Lederhaut hindurch zu ermöglichen. In geringem Abstand von den stirnseitigen Enden sind Ösen oder Bohrungen im Grundkörper angebracht, an denen die Nähte fixiert werden können, welche andererseits außen an der Lederhaut eine weitere Fixierung erhalten. Die Implantation des Implantats im Bereich der sogenannten "Pars plana" an der Innenseite der Lederhaut erlaubt es, mit einem minimalisierten operativen Eingriff auszukommen.

Die Fläche, welche dem als Kugelflächensegment geformten Flächenabschnitt gegenüberliegt, kann ebenfalls als Kugelflächensegment gekrümmt oder mindestens in ihrem mittleren Bereich eben ausgebildet sein.

Die Umfangskontur desjenigen Flächenabschnitts, welche gegen die Innenfläche der Lederhaut anliegt, besitzt - in Aufsicht gesehen - näherungsweise die Form einer Ellipse, besonders bevorzugt ist diese Umfangskontur zu den Enden hin noch stärker als ellipsenförmig verjüngt. Damit das Implantat, welches den Drucksensor, die Telemetrieschaltung und die Sende- und Empfangsspule enthält, an der Parsplana-Stelle mit einem minimalisierten operativen Eingriff eingebracht werden kann, soll das Implantat eine möglichst geringe Größe aufweisen und eine Länge von Ende zu Ende des Grundkörpers aufweisen, für die bevorzugt gilt: 0,5 R < I < 1,5 R, und besonders bevorzugt I = R, wobei R der Radius der Innenfläche der Netzhaut des Auges ist und eine Größe von etwa R = 11 mm aufweist. Die maximale Breite, etwa am Mittenquerschnitt, senkrecht zur Längsachse, ist bevorzugt kleiner als 2 mm und die maximale Dicke des Implantats, gemessen zwischen der zur Anlage an der Lederhaut-Innenfläche kommende Kugeloberflächensegments und der darunter liegenden Innenfläche, beträgt bevorzugt weniger als 3 mm.

Besonders bevorzugt ist die Telemetrieschaltung und der Drucksensor auf einem ersten flexiblen Hilfsträger angeordnet, der in den Grundkörper eingefügt ist bzw. der zusammen mit seinen Bauteilen von dem Grundkörper ummantelt ist. Die Telemetrieschaltung und der Drucksensor sind bevorzugt nebeneinander auf dem ersten Hilfsträger angeordnet, um eine geringe Bauhöhe des Implantats zu realisieren, auf dem ersten Hilfsträger sind Kontaktflächen zum Anschließen der Sendeund Empfangsspule vorgesehen.

Bevorzugt lässt sich die Sende- und Empfangsspule auf dem ersten Hilfsträger anbringen oder um einen Abschnitt des ersten Hilfsträgers herumwickeln, beispielsweise ist zu diesem Zweck ein separater Kern aus ferromagnetischem Material vorgesehen, um den die Windungen der Spule herumlaufen. Alternativ ist auf dem ersten Hilfsträger eine ferromagnetische ebene Schicht aufgebracht, um die herum oder auf der die Wicklungen der Sende- und Empfangsspule verlaufen.

Gemäß einer bevorzugten Ausführungsform der Erfindung ist die Sende- und Empfangsspule auf oder um den Grundkörper herumgewickelt bzw. die Windungen der Spule sind auf dem Grundkörper aufgedampft, um auf diese Weise einen möglichst großen Spulenquerschnitt, und damit eine möglichst große Induktivität zu erzeugen, damit die Reichweite zwischen Implantat und externen Empfangseinrichtungen möglichst groß wird.

Alternativ ist es auch möglich, die Sende- und Empfangsspule auf einen zweiten Hilfsträger aufzubringen, und diesen zweiten Hilfsträger - beispielsweise mit den Spulenanschlüssen - an entsprechenden Anschlussflächen des ersten Hilfsträgers anzuschließen.

Gemäß einer bevorzugten Ausführungsform der Erfindung ist die Sende- und Empfangsspule auf dem zweiten Hilfsträger auf einen ferromagnetischen Kern oder um eine ferromagnetische Beschichtung herum aufgebracht.

Der Grundkörper umschließt bevorzugt den oder die Hilfsträger sowie die Bauelemente, wie Telemetrieschaltung, Drucksensor sowie Sende- und Empfangsspule, wobei die Druckmembran des Drucksensors mit der Augenflüssigkeit direkt bzw. indirekt über ein Koppelelement in Druckkontakt steht. Als Koppelelement lässt sich beispielsweise eine Silikonfüllung verwenden, welche den zur Druckmembran führenden Freiraum ausfüllt. Der Grundkörper lässt sich als Außenhülle um die Bauteile und den Hilfsträger formen, beispielsweise pressen oder gießen. Bei diesem Fertigungsprozeß wird dem Grundkörper die Außenkontur verliehen, welche die spezifische Gestalt des erfindungsgemäßen Implantates ausmacht.

Um eine Langzeit-Biokompatibilität gegenüber der Umgebung im Innenraum des Auges zu gewährleisten, lässt sich bevorzugt das Implantat noch mit einer dünnen Metallschicht, beispielsweise aus Titan oder Titanoxyd, beschichten, deren Dicke jedoch so gering bemessen ist, dass dadurch die elektromagnetische Ankopplung der Telemetrieeinrichtung bzw. Abstrahlung der Telemetriesignale an die externe Empfangseinrichtung nicht wesentlich beeinträchtigt wird.

Vorteilhafte Weiterbildungen der Erfindung sind durch die Merkmale der Unteransprüche gekennzeichnet.

Im folgenden werden Ausführungsbeispiele der Erfindung anhand der Zeichnungen näher erläutert. Es zeigen:
- Figur 1: einen schematischen Querschnitt durch im menschlichen Auge mit einoperiertem Implantat;
- Figur 2a bis 2c: eine Aufsicht, Seitenansicht und einen Querschnitt durch eine erste Ausführungsform des intraokularen Implantats;
- Figur 3a, 3b, 3c: verschiedene Ansichten einer zweiten Ausführungsform des intraokularen Implantats;
- Figur 4: einen Hilfsträger, auf dem ein Drucksensor und eine Telemetrieschaltung angeordnet sind;
- Figur 5: eine weitere Ausführungsform des ersten Hilfsträgers mit Telemetrieschaltung und Drucksensor, an den ein zweiter Hilfsträger angekoppelt ist, der im wesentlichen die Sende- und Empfangsspule enthält;
- Figur 6: eine vergrößerte Darstellung des Grundkörpers gemäß Figur 2b, der die Telemetrieeinrichtung gemäß Figur 5 enthält; und
- Figur 7: ein schematisches Schaltbild der Telemetrieschaltung.

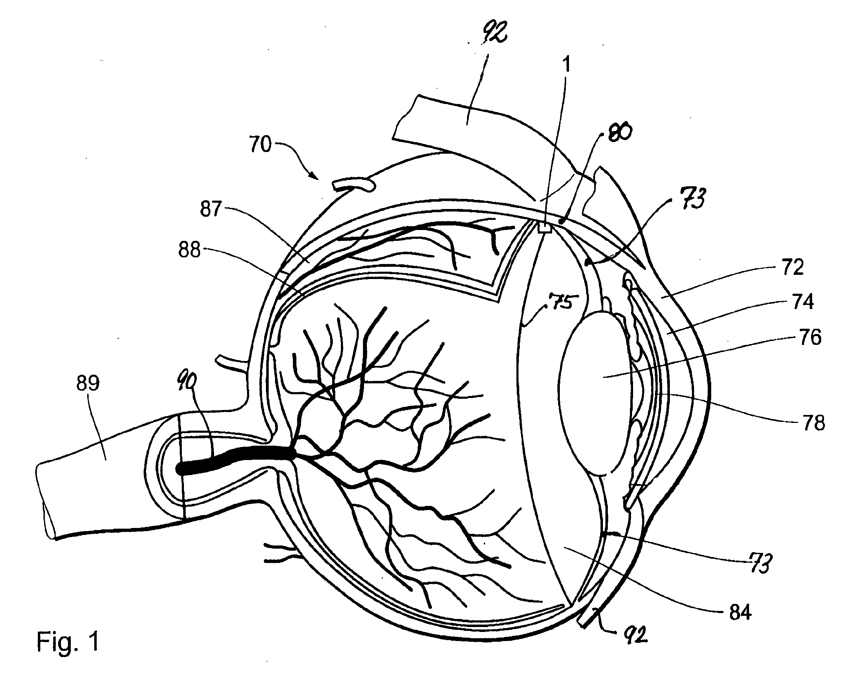

Figur 1 zeigt einen Querschnitt durch das menschliche Auge 70. Hinter der Hornhaut 72 befindet sich die vordere Augenkammer 74, die von der Regenbogenhaut 78 begrenzt ist. Hinter der Regenbogenhaut 78 befindet sich die Linse 76, die an ihrem Rand von den Zonular-Fasern 73 gehalten und an der Innenseite der Lederhaut 78 befestigt ist. Hinter der Linse 76 liegt der Glaskörperraum 84. Im oberen Bereich des kugelförmigen Augenkörpers ist - auf der Innenseite der Lederhaut 80 die Aderhaut 87 und unmittelbar darunterliegend die Netzhaut 88 angeordnet. Auf der Rückseite des Augenkörpers mündet der Sehnerv 89, durch den die Zentralarterie 90 hindurchgeführt ist und die Blutversorgung der in der Aderhaut 87 liegenden Adern übernimmt. Dargestellt ist ferner der Augenmuskel 92, der die Beweglichkeit des Auges sicherstellt. Auf der Innenseite der Lederhaut 80, zwischen dem Ende Zenolar-Fäden 73 und dem vorderen Ende der Netzhaut befindet sich der sogenannten Pars-plana-Bereich, an welchem das Implantat 1 - an der Innenseite der Lederhaut formschlüssig und druckfrei anliegend das Implantat - durch eine entsprechende operative Öffnung der Lederhaut eingeführt und dann an dieser Stelle mittels Fäden an der Lederhaut befestigt ist.
Diese Position des Implantates 1 ermöglicht es, die Operation mittels eine minimalisierten operativen Eingriffes vorzunehmen und dabei die funktionell wichtigen Strukturen des Auges überhaupt nicht zu berühren bzw. nicht zu schädigen. Um auch langfristig Gewebeänderungen oder Störungen zu verhindern, ist die formschlüssige, d.h. druckfreie und spannungsfreie sowie bündige Anlage an der Innenfläche der Lederhaut durch die erfindungsgemäße Formgebung des Implantates wesentlich.

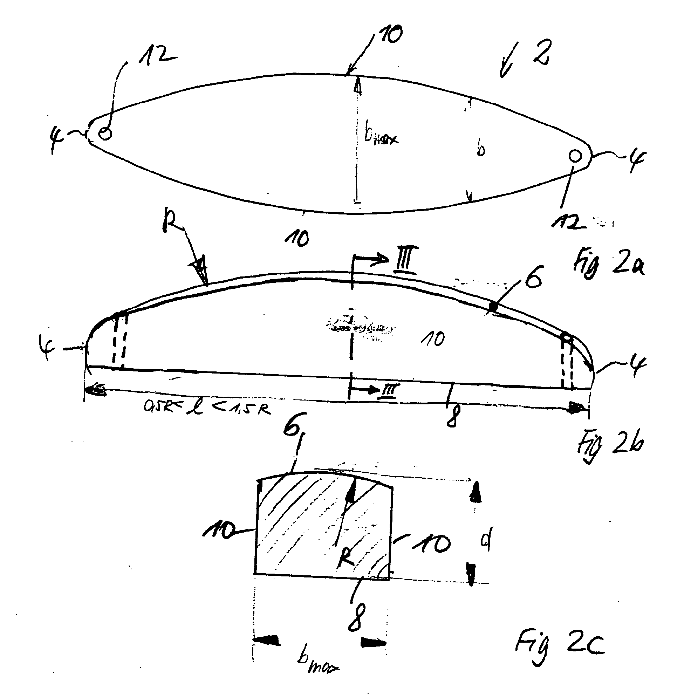

In den Figuren 2a, 2b und 2c ist eine erste Ausführungsform des intraokularen Implantats in Aufsicht (Figur 2a), in Seitenansicht (Figur 2b) und im Querschnitt (Figur 2c) dargestellt. Das Implantat 1 enthält einen Grundkörper 2 aus biokompatiblem Material, der eine längliche Form und in Längsrichtung einander gegenüberliegende Enden 4 besitzt. Die Formgebung des Implantates 1 ist zwischen den Enden 4 durch eine Umfangsfläche gekennzeichnet, die durch eine Anlagefläche 6, einer gegenüberliegenden Basisfläche 8 und zwei Seitenflächen 10 gekennzeichnet ist, vgl. insbesondere Figur 2c. Der als Anlagefläche 6 bezeichnete Abschnitt der Umfangsfläche besitzt die Form eines Kugeloberflächensegments, wobei die Kugeloberfläche einen Radius R besitzt, der näherungsweise dem Krümmungsradius der Innenfläche der Lederhaut des menschlichen Auges entspricht und daher im Bereich zwischen 10 mm und 12 mm, näherungsweise bei 11 mm, liegt. Beim Einoperieren des Implantates in das menschliche Auge kommt die Anlagefläche 6 im Pars-plana-Bereich zur formschlüssigen Anlage an die Innenfläche der Lederhaut, und durch diese Formgebung ist sichergestellt, dass auch über einen langen Zeitraum hinweg keine Gewebestörungen oder -reizungen im Auge auftreten.

Wie insbesondere der Aufsicht gemäß Figur 2a zu entnehmen ist, verjüngt sich der längliche Grundkörper zu seinen stirnseitigen Enden 4 hin, der von einer mittleren Querschnittsebene näherungsweise symmetrisch und weist in geringem Abstand von den stirnseitigen Enden 4 je eine Öse oder eine Bohrung 12 auf, an der die Fixation des zur Befestigung des Implantats 1 erforderlichen Fadens fixiert werden kann. Die der als Kugeloberflächensegment ausgebildeten Anlagefläche 6 gegenüberliegende Basisfläche 8 ist in der dargestellten Ausführungsform eben ausgebildet, sie kann alternativ ebenfalls als Kugeloberflächensegment insbesondere mit dem Radius R ausgebildet und entsprechend gekrümmt sein. Die stirnseitigen Enden 4 des Grundkörpers 2 sind abgerundet, die in Aufsicht auf die Anlagefläche 6 gesehene Umfangskontur 10 weist - in der dargestellten Ausführungsform - gemäß Figur 2a näherungsweise die Form einer Ellipse auf, wobei die Verjüngung zu den Enden 4 hin noch stärker als ellipsenförmig sein kann, um eine besonders einfache Einführung durch die Lederhaut des Auges hindurch in den Augeninnenraum zu ermöglichen.

Die Länge I zwischen den beiden Enden 4 des Implantats 2 beträgt 0,5 R < I < 1,5 R, sie liegt besonders bevorzugt im Bereich von I ≤ R und besitzt somit ein absolutes Maß von < 11 mm. Die maximale Breite bₘₐₓ ist bevorzugt < 3 mm, die maximale Dicke zwischen der Anlagefläche 6 und der Basisfläche 8 soll den Wert dₘₐₓ = 3 mm nicht überschreiten.

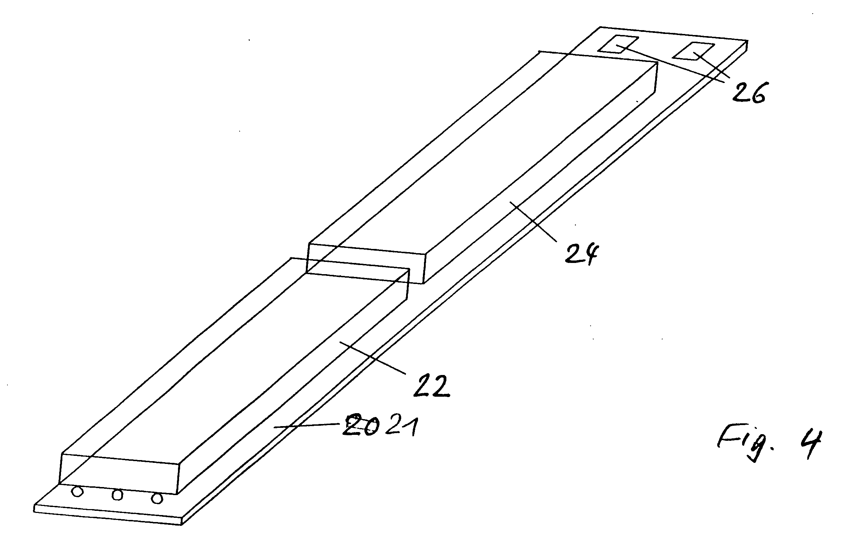

In dem Grundkörper 2 sind Telemetrieeinrichtungen 20 angeordnet, die dazu dienen, den in der Umgebung des Implantates herrschenden Druck, bei implantiertem Implantat also den Augeninnendruck, zu bestimmen und ein entsprechendes elektrisches Telemetriesignal drahtlos an äußere Empfangseinrichtungen abzugeben. Die Telemetrieeinrichtungen sind in den Figuren 4 bis 7 dargestellt und werden in Verbindung mit diesen Figuren näher erläutert.

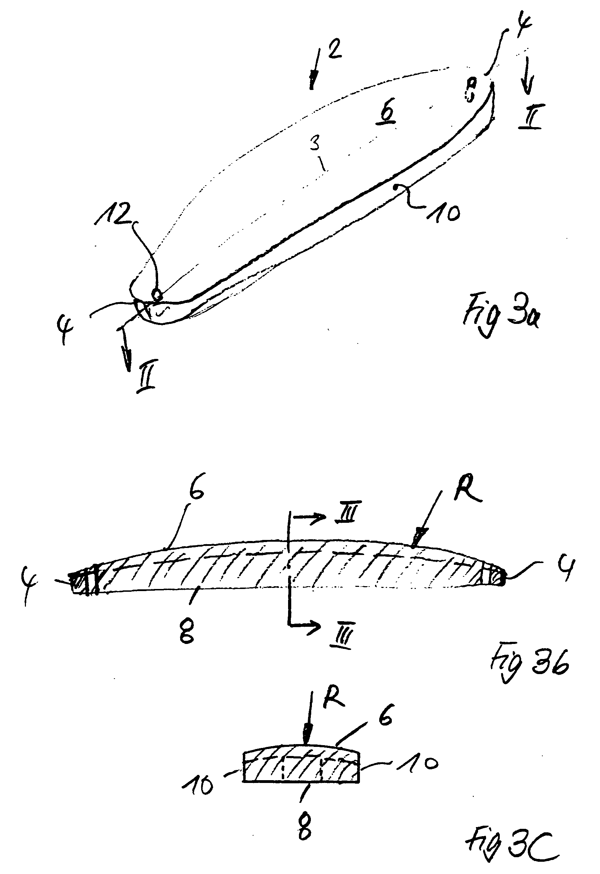

In den Figuren 3a, 3b und 3c ist eine zweite Ausführungsform des Implantats perspektivisch (Figur 3a), im Längsschnitt (Figur 3b) und im Querschnitt (3c) dargestellt. Der längliche Grundkörper 2 besitzt zwei einander in Längsrichtung gegenüberliegende Enden 4, und zwischen den Enden eine Umfangsfläche, die von der Anlagefläche 6, den beiden Seitenflächen 10 und der der Anlagefläche 6 gegenüberliegende Basisfläche 8 gebildet ist. Die Anlagefläche 6 ist als Segment einer Kugeloberfläche mit dem Radius R ausgebildet, ist also in allen Richtungen mit dem Radius R gekrümmt, um eine formschlüssige, druck- und spannungsfreie Anlage an der Innenfläche der Lederhaut des Auges zu gewährleisten. Der Grundkörper 2 verjüngt sich zu den Enden 4 hin und besitzt im Bereich der Enden mindestens je eine Öse oder Bohrung 12 zum Befestigen eines geeigneten Fadens, mit dem das Implantat dann im Pars-plana-Bereich an der Innenfläche der Lederhaut des Auges festgesetzt werden kann. Die Dicke des Grundkörpers 2 ist in der dargestellten Ausführungsform vergleichsweise gering bemessen, die übrigen Abmessungen entsprechen weitgehend der Ausführungsform gemäß Figur 2.

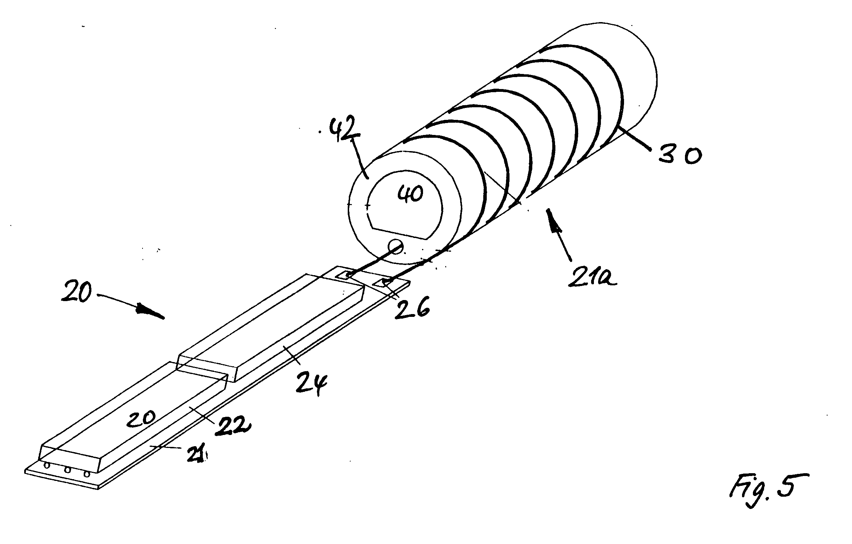

In den Figuren 4 und 5 ist eine erste Ausführungsform der Telemetrieeinrichtungen 20 vorgesehen, die in dem Grundkörper 2 vorgesehen sind, wobei der Grundkörper die Bauteile der Telemetrieeinrichtungen als Außenhülle umgeben kann und beispielsweise um die Telemetrieeinrichtungen herum geformt, gepresst oder gegossen ist.

Die Figuren 4 und 5 zeigen einen ersten Hilfsträger 21, auf dem ein Drucksensor 22 angeordnet ist, der mittels einer Druckmembran (nicht dargestellt) mit seiner Umgebung in Verbindung steht und den Druck des umgebenden Fluids, beispielsweise - bei implantiertem Implantat - den Augeninnendruck ermittelt und ein diesen Druck kennzeichnendes elektrisches Sensorsignal abgibt. Auf dem ersten Hilfsträger 21 ist ferner eine Telemetrieschaltung 24 angeordnet, die elektrisch mit dem Drucksensor verbunden ist und aus dem Sensorsignal ein den Augeninnendruck kennzeichnendes elektrisches Telemetriesignal erzeugt. Auf dem ersten Hilfsträger 21 sind Anschlußkontakte 26 vorgesehen, an denen eine Sende- und Empfangsspule 30 anschließbar ist, die beispielsweise auf einen zylindrisch zweiten Hilfsträger 21a gewickelt ist und zum drahtlosen Empfang von Speisesignalen dient, welche der Telemetrieschaltung 24 ausreichend Speiseenergie zuführt, und welche zur drahtlosen Abgabe des elektromagnetischen Telemetriesignals an externe Empfangseinrichtungen dient. Der zweite Hilfsträger 21a kann als flexibler Kunststoffkörper oder als ferromagnetischer Kern 40 mit gegebenenfalls einer elektrischen Isolierschicht 42 ausgebildet sein, um die Induktivität der Sende- und Empfangsspule 30 entsprechend zu erhöhen.

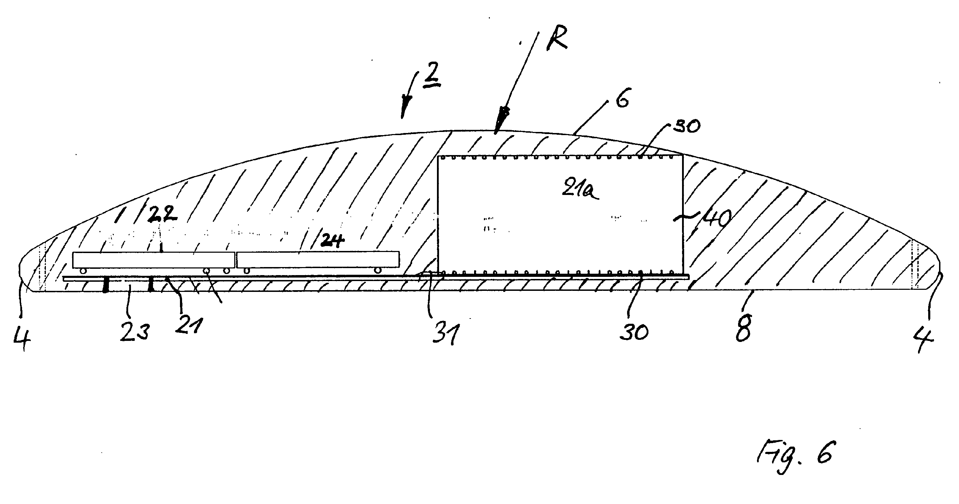

Figur 6 zeigt eine vergrößerte Darstellung des Implantats der Ansicht nach Figur 2b, wobei das Implantat die Telemetrieeinrichtungen gemäß Figur 5 enthält. Der Grundkörper 2 ist in einem Schnitt dargestellt und weist die Anlagefläche 6 auf, die mit dem Krümmungsradius R kugelförmig gekrümmt ist. Der Anlagefläche 6 gegenüber liegt die Basisfläche 8, der Grundkörper 2 besitzt in Längsrichtung zwei einander gegenüberliegende stirnseitige Enden 4. Der Grundkörper 2 ist um Telemetrieeinrichtungen 20 herum gepresst, die auf einem ersten Hilfsträger 21 aufgebaut sind und aus einem Drucksensor 22 sowie der Telemetrieschaltung 24 bestehen, die auf dem Hilfsträger 21 mittels geeigneter Kontakte 23 auf dem Hilfsträger 21 angeordnet sind. Außerdem ist die Sende- und Empfangsspule 30 auf einem zweiten Hilfsträger 21a angeordnet, und dieser zweite Hilfsträger 21a ist - in der dargestellten Ausführungsform - auf dem ersten Hilfsträger 21 angeordnet und dort befestigt. Der zweite Hilfsträger 21a kann beispielsweise als zylindrischer Kunststoffkörper oder als Ferritkern ausgebildet sein. Die Sende- und Empfangsspule 30 ist über Anschlüsse 31 mit der Telemetrieschaltung 24 verbunden. Unterhalb des Drucksensors 22, und zwar unterhalb seiner Druckmembran, befindet sich im Grundkörper 2 und im ersten Hilfsträger 21 ein Freiraum 23, durch den das Umgebungsmedium - bei implantiertem Implantat also die Augenflüssigkeit - mit der Druckmembran des Drucksensors 22 in Verbindung steht.

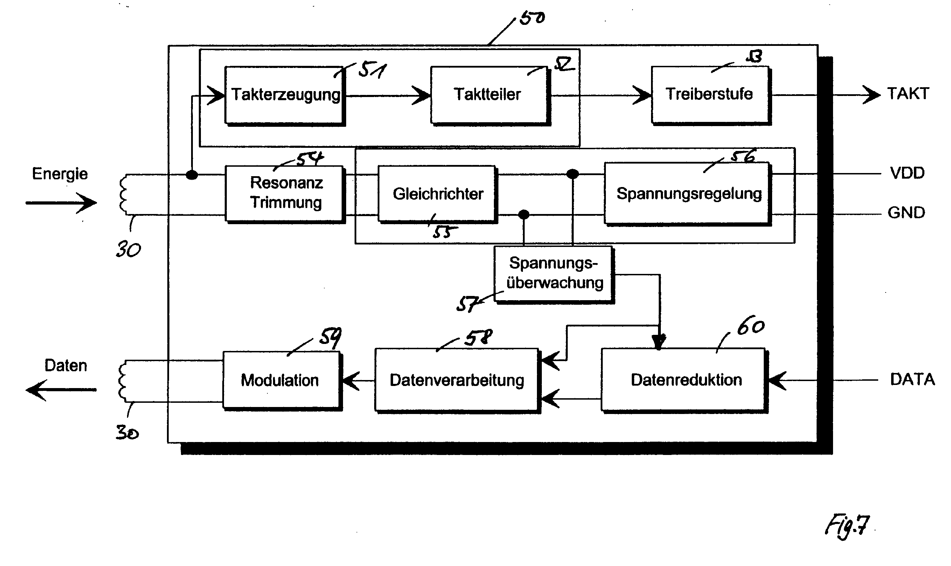

Figur 7 zeigt einen Querschnitt durch eine weitere Ausführungsform. Figur 7 zeigt ein schematisches Schaltbild der zum Einsatz gelangenden Telemetrieschaltung. Von der Sende- und Empfangsspule 30 wird die von einer externen Speiseschaltung abgestrahlte Speiseenergie empfangen und über eine Takterzeugung 52 und einen Taktteiler 52 einer Treiberstufe 53 zugeführt, die einen erforderlichen elektrischen Takt mit gewünschter Frequenz und Amplitude abgibt und der weiteren Schaltung zur Verfügung stellt. Die Speiseenergie wird ferner über eine Resonanztrimmung 54 und einen Gleichrichter 55 an eine Spannungsüberwachung 57 abgegeben, die mit einer Spannungsregelung 56 verbunden ist und eine geregelte Spannung abgibt.

Von dem Drucksensor (nicht dargestellt) wird das Sensorsignal am Eingang "data" zugeführt und mittels einer geeigneten Datenreduktionsschaltung 60 an eine Datenverarbeitung 58 weitergegeben, welche die Daten über eine Modulationsschaltung 59 der Sende- und Empfangsspule 30 zuführt. Die auf diese Weise bearbeiteten Sensorsignale, welche den Augeninnendruck kennzeichnen, werden von der Sendeund Empfangsspule 30 elektromagnetisch abgestrahlt und von äußeren Empfangseinrichtungen, die sich außerhalb des menschlichen Körpers befinden, bearbeitet und ausgewertet und - nach entsprechender Kalibrierung - weiter verarbeitet oder angezeigt. Die Datenreduktion 60, die Datenverarbeitung 58 und gegebenenfalls auch die Modulationsschaltung 59 erhalten ihre Versorgungsspannung von der Spannungsregelung 56, die ihrerseits ihre Speiseenergie telemetrisch über die Spule 30 und die Schaltungen 51 bis 57 von einer externen HF-Quelle erhält.

## Patentansprüche

1. Intraokulares Implantat zurtelemetrischen Bestimmung des Innendrucks des menschlichen Auges, mit einem Drucksensor (22) zur Abgabe eines den Augeninnendruck kennzeichnenden elektrischen Sensorsignals,
einer Telemetrieschaltung (24) zur Erzeugung eines den Augeninnendruck kennzeichnenden elektrischen Telemetriesignales aus dem Sensorsignal,
einer Sende- und Empfangsspule (30) zum drahtlosen Empfang von Speisesignalen, welche der Telemetrieschaltung (24) ausreichend Speiseenergie zuführen, und zur drahtlosen Abgabe des Telemetriesignales an externe Empfangseinrichtungen, und
einem Grundkörper (2) aus einem biokompatiblen Material, der den Drucksensor (22), die Telemetrieschaltung (24) und die Sende- und Empfangsspule (30) trägt,
**dadurch gekennzeichnet, dass** der Grundkörper (2) eine längliche Form besitzt, und dass ein Flächenabschnitt (6) der sich zwischen den in Längsrichtung einander gegenüberliegenden Enden (4) des Grundkörpers erstreckt und in Längsrichtung mit einem Radius R gekrümmt ist, wobei R näherungsweise dem Krümmungsradius der Innenfläche der Lederhaut des menschlichen Auges entspricht.

2. Intraokulares Implantat nach Anspruch 1,
**dadurch gekennzeichnet, dass** der Flächenabschnitt (6) der Umfangsfläche (6, 8, 10) in Form eines Kugeloberflächensegments gekrümmt ist und einen Krümmungsradius R aufweist, der näherungsweise dem Krümmungsradius der Innenfläche der Lederhaut des menschlichen Auges entspricht.

3. Intraokulares Implantat nach Anspruch 1 oder 2,
**dadurch gekennzeichnet, dass** der Krümmungsradius R zwischen 10 und 12 mm liegt.

4. Intraokulares Implantat nach Anspruch 3,
**dadurch gekennzeichnet, dass** der Krümmungsradius R näherungsweise 11 mm beträgt.

5. Intraokulares Implantat nach einem der vorstehenden Ansprüche,
**dadurch gekennzeichnet, dass** sich der längliche Grundkörper (2) zu seinen stirnseitigen Enden (4) hin im Querschnitt verjüngt.

6. Intraokulares Implantat nach Anspruch 5,
**dadurch gekennzeichnet, dass** der längliche Grundkörper (2) stabförmig ausgebildet ist und sich zu beiden Enden (4) hin von einer mittigen Querschnittsebene hin näherungsweise symmetrisch verjüngt.

7. Intraokulares Implantat nach einem der vorstehenden Ansprüche,
**dadurch gekennzeichnet, dass** der Grundkörper (2) in geringem Abstand von den stirnseitigen Enden (4) Ösen oder Bohrungen (12) zur Nahtfixation enthält.

8. Intraokulares Implantat nach einem der vorstehenden Ansprüche,
dass die Fläche (8), welche dem als Kugeloberflächensegment geformten Flächenabschnitt (6) gegenüberliegt, ebenfalls als Kugeloberflächensegment gekrümmt ist oder mindestens in ihrem mittleren Bereich eben ausgebildet ist.

9. Intraokulares Implantat nach einem der vorstehenden Ansprüche,
**dadurch gekennzeichnet, dass** die stirnseitigen Enden (4) abgerundet sind.

10. Intraokulares Implantat nach einem der vorstehenden Ansprüche,
**dadurch gekennzeichnet, dass** die in Aufsicht gesehene Umfangskontur (10) desjenigen Flächenabschnitts (6), der in Form eines Kugelflächensegmentes dem Radius R gekrümmt ist, näherungsweise die Form einer Ellipse aufweist.

11. Intraokulares Implantat nach Anspruch 10,
**dadurch gekennzeichnet, dass** die in Aufsicht gesehene Umfangskontur (10) des Flächenabschnitts (6), der in Form eines Kugelflächensegments gekrümmt ist, zu den Enden (4) sich stärker als ellipsenförmig verjüngt.

12. Intraokulares Implantat nach einem der vorstehenden Ansprüche,
**dadurch gekennzeichnet, dass** die Länge I zwischen den beiden Enden (4) 0,5 R < I < 1,5 R beträgt, wobei R der Krümmungsradius der Innenfläche der Lederhaut des menschlichen Auges entspricht.

13. Implantat nach Anspruch 12,
**dadurch gekennzeichnet, dass** die Länge I zwischen den Enden (4) näherungsweise I = R ist.

14. Intraokulares Implantat nach einem der vorstehenden Ansprüche,
**dadurch gekennzeichnet, dass** die zwischen den beiden Enden (4) gemessene Länge I des Grundkörpers I < 12 mm ist, die maximale Breite bₘₐₓ < 3 mm und die maximale Dicke, gemessen zwischen der Anlagefläche (6) und der Basisfläche (8) dₘₐₓ < 3 mm beträgt.

15. Intraokulares Implantat nach einem der vorstehenden Ansprüche,
**dadurch gekennzeichnet, dass** die Telemetrieschaltung (24) und der Drucksensor (22) auf einem ersten Hilfsträger (21) angeordnet sind, der in den Grundkörper (2) eingefügt ist.

16. Intraokulares Implantat nach einem der vorstehenden Ansprüche,
**dadurch gekennzeichnet, dass** die Telemetrieschaltung (24) und der Drucksensor (22) nebeneinander auf dem ersten Hilfsträger (21) angeordnet sind, und dass auf dem ersten Hilfsträger (21) Kontaktflächen (26) zum Anschließen der Sende- und Empfangsspule (30) vorgesehen sind.

17. Intraokulares Implantat nach einem der vorstehenden Ansprüche,
**dadurch gekennzeichnet, dass** die Sende- und Empfangsspule (30) auf dem ersten Hilfsträger (21) angeordnet ist.

18. Intraokulares Implantat nach einem der vorstehenden Ansprüche,
**dadurch gekennzeichnet, dass** die Sende- und Empfangsspule (30) um einen Abschnitt (6) des ersten Hilfsträgers (21) gewickelt ist, der eine Beschichtung oder einen separaten Kern (40) aus ferromagnetischem Material aufweist.

19. Intraokulares Implantat nach einem der Ansprüche 13 bis 18,
**dadurch gekennzeichnet, dass** auf dem ersten Hilfsträger (21) ein ferromagnetisches Material aufgebracht ist, um welches herum die Wicklungen der Sende- und Empfangsspule (30) verlaufen.

20. Intraokulares Implantat nach einem der Ansprüche 13 bis 19,
**dadurch gekennzeichnet, dass** die Sende- und Empfangsspule (30) auf oder um den Grundkörper (2) gewickelt ist.

21. Intraokulares Implantat nach einem der vorstehenden Ansprüche,
**dadurch gekennzeichnet, dass** die Sende- und Empfangsspule (30) auf einem zweiten Hilfsträger (21a) angeordnet ist.

22. Implantat nach Anspruch 21,
**dadurch gekennzeichnet, dass** der zweite Hilfsträger (21a) einen Kern (40) aus ferromagnetischem Material enthält, um den die Wicklungen der Sende- und Empfangsspule (30) herum verlaufen.

23. Implantat nach Anspruch 21 oder 22,
**dadurch gekennzeichnet, dass** zwischen dem Kern (40) des zweiten Hilfsträgers (21a) und den Wicklungen der Sende- und Empfangsspule (30) eine Isolationsschicht vorgesehen ist, und dass die Sende- und Empfangsspule (30) aus einem unisolierten Draht gewickelt ist.

24. Implantat nach einem der vorstehenden Ansprüche,
**dadurch gekennzeichnet, dass** der zweite Hilfsträger (21a) mit dem ersten Hilfsträger (21) elektrisch und/oder mechanisch verbunden ist.

25. Implantat nach einem der vorstehenden Ansprüche,
**dadurch gekennzeichnet, dass** der bzw. die Hilfsträger (21, 21a) in einem Hohlraum des Grundkörpers (2) eingelassen oder von dem Grundkörper (2) eng ummantelt sind.

26. Implantat nach einem der vorstehenden Ansprüche,
**dadurch gekennzeichnet, dass** der Grundkörper (2) einen den Außenraum mit der Druckmembran des Drucksensors (22) verbindenden Freiraum (23) aufweist.

27. Implantat nach Anspruch 26,
**dadurch gekennzeichnet, dass** der Freiraum (23) vor der Druckmembran des Drucksensors (22) mit einem Koppelelement, beispielsweise aus Silikon, gefüllt ist.

28. Implantat nach einem der vorstehenden Ansprüche,
**dadurch gekennzeichnet, dass** der Grundkörper (2), der oder die Hilfsträger (21 bzw. 21a) mit der Telemetrieschaltung (24), dem Drucksensor (22) und der Sendeund Empfangsspule (30) eine Beschichtung aus biokompatiblem Material besitzt.

29. Implantat nach einem der vorstehenden Ansprüche,
**dadurch gekennzeichnet, dass** der Grundkörper (2) und/oder die im Grundkörper (2) enthaltenen Bauteile mit einer Außenbeschichtung aus biokompatiblem Material eingekapselt oder eingegossen sind.

30. Implantat nach einem der vorstehenden Ansprüche,
**dadurch gekennzeichnet, dass** der Grundkörper (2) als Außenhülle um die Bauteile wie Telemetrieschaltung (24), Drucksensor (22), Sende- und Empfangsspule (30) geformt, gepresst oder gegossen ist.

31. Implantat nach einem der vorstehenden Ansprüche,
**dadurch gekennzeichnet, dass** der Rundkörper mit einer dünnen Metallschicht aus biokompatiblem Material, beispielsweise Titan oder Titanoxyd (), beschichtet ist.
